# EUROPÄISCHE PATENTSCHRIFT

(11) **EP 1 505 915 B1**
(45) Veröffentlichungstag und Bekanntmachung des Hinweises auf die Patenterteilung: **07.09.2005**
(21) Anmeldenummer: 03727378.6
(22) Anmeldetag: 26.04.2003
(51) Int. Cl.: A61B 17/32, B26B 13/28

(54) **SCHERE FÜR MEDIZINISCHE ZWECKE**
MEDICAL SCISSORS
CISEAUX A USAGE MEDICAL

(30) Priorität: 07.05.2002 DE 10221321
(43) Veröffentlichungstag der Anmeldung: 16.02.2005
(73) Patentinhaber: Aesculap AG & Co. KG, 78532 Tuttlingen (DE)
(72) Erfinder: DWORSCHAK, Manfred, 78589 Dürbheim (DE); LUTZE, Theodor, 78582 Balgheim (DE); MORALES, Pedro, 78532 Tuttlingen-Nendingen (DE); WEISSHAUPT, Dieter, 78194 Immendingen (DE)
(74) Vertreter: Boehme, Ulrich
(86) Internationale Anmeldenummer: PCT/EP2003/004378
(87) Internationale Veröffentlichungsnummer: WO 2003/094756

(56) Entgegenhaltungen:
- EP-A- 1 066 798
- DE-A- 1 703 255
- DE-U- 20 113 020
- GB-A- 1 279 003
- US-A- 2 239 851
- US-A- 5 320 636

## Beschreibung

Die Erfindung betrifft eine Schere für medizinische Zwecke mit zwei in einem Schluß schwenkbar aneinander gelagerten, jeweils ein Scherenblatt umfassenden Scherenteilen, wobei die beiden Scherenteile oder fest mit ihnen verbundene Teile Spannelemente aufweisen, die beim Verschwenken der Scherenteile aneinander anliegend relativ zueinander bewegt werden und die so geformt sind, daß die beiden Scherenteile beim Schließen aus einer Ruhestellung, in der die beiden Scherenblätter in Richtung der Schwenkachse nicht oder nur wenig gegeneinander gespannt sind, in eine Spannstellung gelangen, in der die beiden Scherenblätter in Richtung ihrer Schwenkachse einander angenähert und dadurch gegeneinander gespannt werden, wobei die Spannelemente eine gegenüber einer senkrecht auf der Schwenkachse stehenden Verschwenkebene geneigte bahnförmige Stützfläche und einen daran anliegenden Bahnfolger umfassen.

Im medizinisch-chirurgischen Bereich werden Scheren in großem Umfange benötigt, um Gewebeteile, Nahtmaterial, Verbandstoff etc. zuverlässig zu schneiden. Dabei ist es außerordentlich wichtig, daß die Schneiden der Schere beim Schneidvorgang im Kontaktbereich der Schneiden zuverlässig aneinander anliegen, und zwar möglichst unter Vorspannung.

Bei herkömmlichen Scheren aus Metall ist dies durch eine entsprechende Formgebung der Scherenteile zu erreichen, Schwierigkeiten ergeben sich aber, wenn die Scheren aus Kunststoff gefertigt werden, da die Kunststoffe in der Regel Kriechverhalten zeigen, das heißt, das Material weicht aus, wenn die Teile längere Zeit gegeneinander gedrückt werden. Wenn eine solche Schere lagert, besteht daher die Gefahr, daß das Material so stark verformt wird, daß ein zuverlässiger Schneidvorgang nicht mehr möglich ist.

In der US 2,239,851 A ist eine metallische Schere beschrieben, bei welcher die beiden Scherenteile durch eine Blattfeder gegeneinander gespannt werden, diese Blattfeder drückt eine Kugel gegen den anderen Scherenteil und führt dadurch zu der gewünschten Vorspannung der Schneiden. Allerdings ist dies eine recht komplizierte Konstruktion, bei der zusätzliche Einzelteile notwendig sind. Für die Herstellung von Scheren aus Kunststoff ist diese Konstruktionsmöglichkeit nicht zu verwenden, da Kunststoffblattfedern mit der notwendigen Federkraft nicht zur Verfügung stehen.

Es ist Aufgabe der Erfindung, Scheren der gattungsgemäßen Art so auszubilden, daß durch einen relativ einfachen Aufbau gewährleistet ist, daß die Scherenteile im Bereich der Scherenblätter immer zuverlässig gegeneinander gespannt sind und aneinander anliegen, auch wenn die Schere längere Zeit gelagert ist.

Diese Aufgabe wird bei einer Schere der eingangs beschriebenen Art erfindungsgemäß dadurch gelöst, daß die bahnförmige Stützfläche am Rande einer Lageröffnung eines Scherenteils und der Bahnfolger an einer diese Lageröffnung durchsetzenden Lagerwelle des anderen Scherenteils angeordnet ist.

Bei einer anderen bevorzugten Ausgestaltung der Erfindung wird diese Aufgabe erfindungsgemäß dadurch gelöst, daß der Bahnfolger am Rande einer Lageröffnung eines Scherenteils und die bahnförmige Stützfläche an einer diese Lageröffnung durchsetzenden Lagerwelle des anderen Scherenteils angeordnet sind.

Durch diese Ausgestaltung wird dafür Sorge getragen, daß die Scherenteile der Schere in ihrer geöffneten Stellung nicht oder nur geringfügig gegeneinander gespannt sind, so daß keine Gefahr besteht, daß durch Kriechen des Materials eine Verformung erfolgt. Andererseits wird beim Schließen der Scherenteile durch die Spannelemente dafür Sorge getragen, daß die beiden Scherenteile in Richtung der Lagerwelle gegeneinander gespannt werden, und dies führt zwangsläufig dazu, daß die Scheren blätter gegeneinander gespannt werden, im Anlagebereich der Scherenblätter gleiten diese also immer zuverlässig und unter Vorspannung aneinander entlang, so daß ein einwandfreier Schneidvorgang gewährleistet ist. Es ist vorteilhaft, eine solche Schere mit geöffneten Scherenteilen aufzubewahren, dadurch ist sichergestellt, daß auch bei kriechendem Material keine unerwünschte Verformung eintritt, im Einsatz werden dann die Scherenteile in die Schließstellung verschwenkt und durch die beschriebenen Spannelemente durch Gegeneinanderspannen funktionsfähig gemacht.

Besonders günstig ist es dabei, wenn der Bahnfolger vorgewölbt ausgebildet ist, so daß er punkt- oder linienförmig an der Stützfläche anliegt. Diese Vorwölbung kann in Form einer Zylinderfläche erfolgen, aber auch in Form einer Kugelteilfläche, bei einer Zylinderfläche ergibt sich im wesentlichen eine linienförmige Anlage, bei einer kugeligen Ausbildung eine punktförmige Anlage an der Stützfläche.

Eine besonders bevorzugte Ausführungsform ergibt sich, wenn vorgesehen wird, daß ein Scherenteil eine unrunde Lageröffnung aufweist, durch die eine Lagerwelle des anderen Scherenteils hindurchragt, daß die Lagerwelle an ihrem dem anderen Scherenteil abgewandten Ende eine kopfförmige unrunde Verbreiterung trägt, die bei geöffneten Scherenteilen durch die unrunde Lageröffnung hindurchpaßt und die beim Verschwenken der Scherenteile in Schließrichtung mit ihrer Unterseite den Rand der Lageröffnung überdeckt und damit ein Herausgleiten der Lagerwelle aus der Lageröffnung verhindert. Die Lagerwelle und die Lageröffnung bilden also eine Bajonettverriegelung, die in einer bestimmten Winkelstellung ein Einführen der Lagerwelle in die Lageröffnung erlaubt, in anderen Winkelstellungen dagegen nicht.

Dabei ist es besonders günstig, wenn der Rand der Lageröffnung und die Unterseite der Verbreiterung die beiden Spannelemente ausbilden.

Vorzugsweise können an der Lageröffnung und der Lagerwelle diametral einander gegenüberliegend je zwei bahnförmige Stützflächen beziehungsweise Bahnfolger angeordnet sein, so daß die die beiden Scherenteile gegeneinander spannenden Kräfte symmetrisch auf beiden Seiten des Schlusses angreifen, eine Verklemmung der beiden Scherenteile wird dadurch verhindert.

Die Neigung der bahnförmigen Stützfläche gegenüber der Verschwenkebene kann beispielsweise zwischen ½° und 3° liegen, es genügen also hier relativ geringe Neigungen, um die gewünschte Vorspannung bei der Schließbewegung zu erzeugen.

Bei einer besonders bevorzugten Ausführungsform ist zusätzlich vorgesehen, daß die Lageröffnung und/oder die Lagerwelle elastische Rastelemente tragen, die bei geöffneten Scherenteilen durch elastische Verformung ein Einschieben der Lagerwelle in die Lageröffnung ermöglichen, die jedoch nach dem Einschieben der Lagerwelle in die Lageröffnung elastisch entspannt ein Herausziehen der Lagerwelle aus der Lageröffnung verhindern oder erschweren. Mit anderen Worten ergibt sich eine Schnappverbindung in Längsrichtung der Lagerwelle, so daß auch bei geöffneten Scherenteilen verhindert wird, daß die Scherenteile nach dem Zusammenstecken wieder auseinanderfallen. In der geöffneten Stellung paßt die Verbreiterung gerade durch die unrunde Lageröffnung, ohne die Schnappverbindung könnten sich die beiden Scherenteile daher unbeabsichtigt wieder lösen.

Günstig ist es, wenn die Rastelemente als seitliche Vorsprünge an der Verbreiterung der Lagerwelle ausgebildet sind. Vorzugsweise tragen die Vorsprünge seitliche Aufgleitflächen, an die sich ein Rücksprung anschließt. Die Aufgleitflächen erleichtern das Einschieben der Lagerwelle in die Öffnung, sobald die Lagerwelle vollständig in die Lageröffnung eingeschoben ist, können sich die Rastelemente wieder entspannen, dann greift der Rand der Lageröffnung in den Rücksprung und verhindert oder erschwert ein Herausziehen der Lagerwelle aus der Lageröffnung.

Durch die beschriebenen Spannelemente werden die beiden Scherenteile beim Schließen derselben so gegeneinander gespannt, daß die Scherenblätter der Scherenteile in ihrem Kontaktbereich gegeneinander gespannt werden.

Die Spannung im Schneidbereich kann ferner dadurch erhöht werden, daß mindestens eines der beiden Scherenteile im Bereich seines Scherenblattes elastisch ausgebildet ist und daß mindestens eines der beiden Scherenteile aus der Verschwenkebene herausgebogen ist. Die beiden Scherenteile werden dadurch auch im Kontaktbereich kräftig gegeneinander gespannt, und zwar ergibt sich dieses Gegeneinanderspannen durch die unterschiedliche Krümmung der Scherenblätter, beispielsweise kann eines gerade und eines gekrümmt sein, und die elastische Verformung mindestens eines der beiden Scherenteile beim Schließen. Dieser Effekt unterstützt den Effekt des Zusammenspannens, der durch die Spannelemente beim Schließen erzeugt wird.

Diese Spannung im Schneidbereich kann auch dadurch erhöht werden, daß die Scherenteile im Bereich ihrer Scherenblätter elastisch ausgebildet und aus der Verschwenkebene herausgebogen sind und daß der Biegeradius des inneren Scherenteils größer ist als der des äußeren Scherenteils, insbesondere ist der Biegeradius des inneren Scherenteils 10 bis 30 % größer als der des äußeren Scherenteils. Das äußere Scherenteil und das innere Scherenteil werden dadurch ebenfalls im Kontaktbereich kräftig gegeneinander gespannt, und zwar ergibt sich dieses Gegeneinanderspannen durch die unterschiedlichen Radien und die elastische Verformung der beiden Scherenteile beim Schließen. Dieser Effekt unterstützt den Effekt des Zusammenspannens, der durch die Spannelemente beim Schließen erzeugt wird.

Die nachfolgende Beschreibung bevorzugter Ausführungsformen der Erfindung dient im Zusammenhang mit der Zeichnung der näheren Erläuterung. Es zeigen:
- Figur 1:: eine Schere mit den beiden Scherenteilen in der Öffnungsstellung;
- Figur 2:: eine Schnittansicht längs Linie 2-2 in Figur 1;
- Figur 3:: eine Draufsicht auf den Schlußbereich der Schere der Figur 1 bei in Öffnungsstellung stehenden Scherenteilen;
- Figur 4:: eine Draufsicht auf den vorderen Bereich der Schere der Figur 1 mit den Scherenteilen in Schließstellung;
- Figur 5:: eine Ansicht ähnlich Figur 3 mit den beiden Scherenteilen in Schließstellung;
- Figur 6:: eine Schnittansicht längs Linie 6-6 in Figur 5 und
- Figur 7:: eine Seitenansicht der beiden Scherenteilen der Schere der Figur 1.

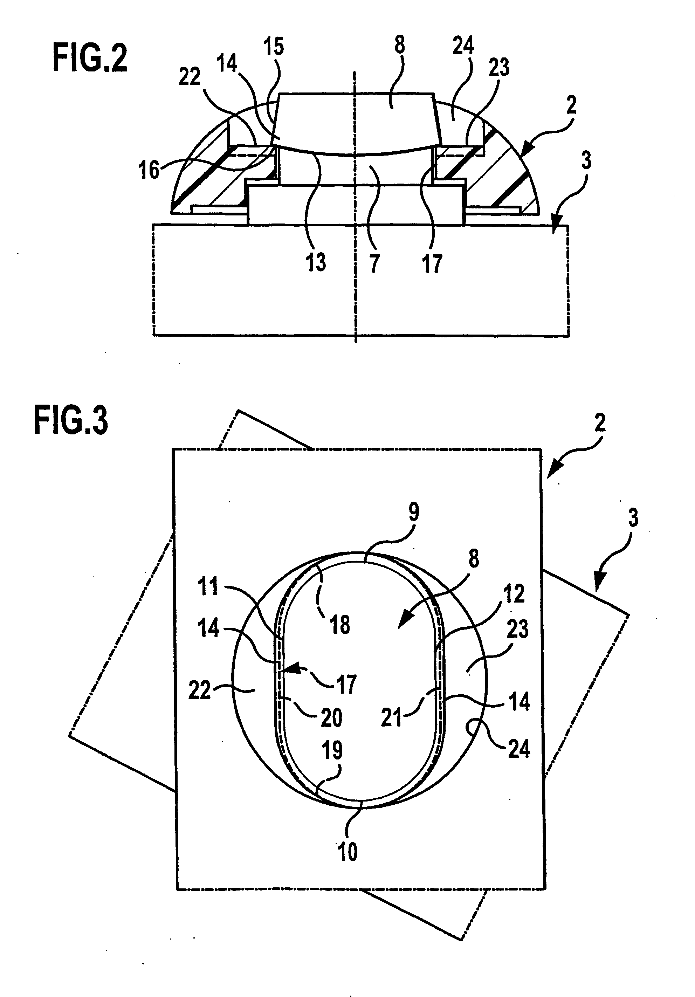

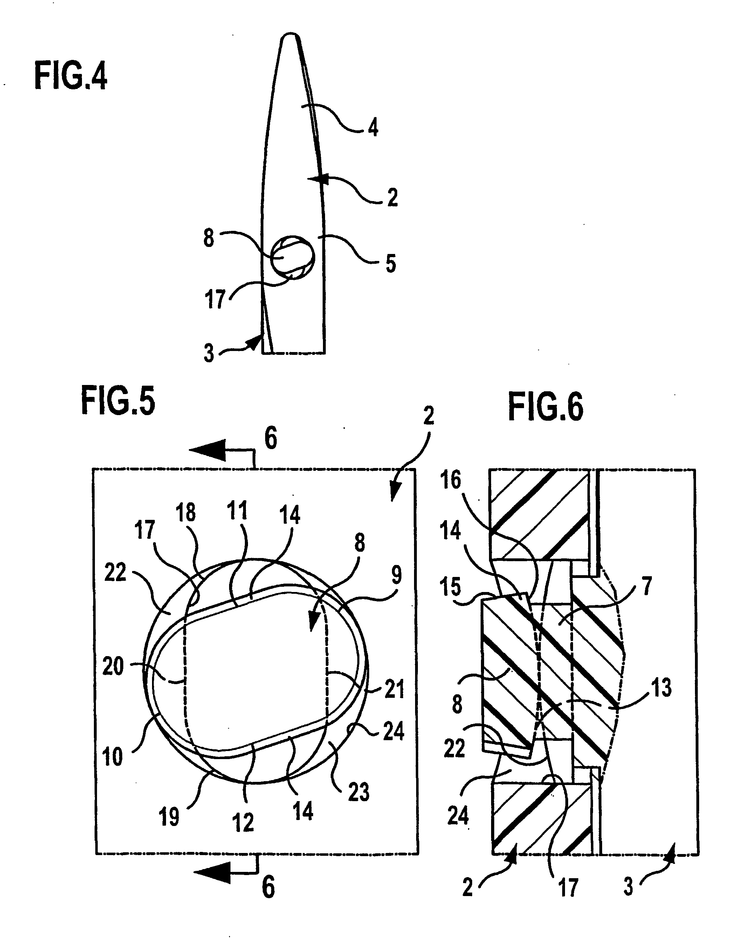

Die in der Zeichnung dargestellte Schere 1 umfaßt zwei Scherenteile 2, 3 mit jeweils einem eine Schneide bildenden Scherenblatt 4, einem Lagerbereich 5 und einem Griffteil 6. Im Lagerbereich 5 sind die beiden Scherenteile 2 und 3 schwenkbar miteinander verbunden. Dazu trägt ein männliches Scherenteil 3 eine von ihm abstehende Lagerwelle 7, die an ihrem freien Ende mit einer kopfförmigen Verbreiterung 8 versehen ist. Diese ist im dargestellten Ausführungsbeispiel im wesentlichen oval ausgebildet, das heißt die Verbreiterung 8 weist an gegenüberliegenden Enden kreisbogenförmige Endabschnitte 9, 10 auf, die sich jeweils über 180° erstrecken, und diese Endabschnitte 9, 10 sind durch geradlinige Abschnitte 11, 12 miteinander verbunden (Figuren 3 und 5). Die beiden geradlinigen Abschnitte 11 und 12 haben einen Abstand voneinander, der etwa dem Durchmesser der Lagerwelle 7 entspricht, die kreisbogenförmigen Endabschnitte 9, 10 stehen dagegen über den Querschnitt der Lagerwelle 7 hervor. Ihre Unterseite 13 ist nach unten tonnenförmig oder kreiszylindrisch vorgewölbt, die Längsachse dieser Vorwölbung fällt dabei mit der Längsachse der Verbreiterung 8 zusammen (Figur 2).

An den beiden geradlinigen Abschnitten 11, 12 sind jeweils Rastvorsprünge 14 angeordnet, die über den Querschnitt der Lagerwelle 7 geringfügig hervorstehen und die eine vom freien Ende der Lagerwelle 7 in Richtung auf das Scherenteil 3 schräg ansteigende Aufgleitfläche 15 und einen daran anschließenden Rücksprung 16 aufweisen, dieser wird durch die Unterseite 13 gebildet, die sich bis an die Aufgleitfläche 15 erstreckt (Figur 2).

In dem anderen, dem sogenannten weiblichen Scherenteil 2 ist im Lagerbereich 5 eine durchgehende Lageröffnung 17 angeordnet, deren Querschnitt im wesentlichen dem Querschnitt der Verbreiterung 8 der Lagerwelle 7 entspricht, sie weist also ebenfalls zwei sich über jeweils 180° erstreckende kreisbogenförmige Endabschnitte 18, 19 auf, die durch geradlinige Abschnitte 20, 21 miteinander verbunden sind. Die Abmessungen der Lageröffnung 17 sind so gewählt, daß die Verbreiterung 8 durch die Lageröffnung 17 hindurchpaßt, wenn die langen Achsen der Lageröffnung 17 und der Verbreiterung 8 miteinander ausgerichtet sind, allerdings werden beim Hindurchschieben die Rastvorsprünge 14 elastisch zusammengedrückt, sie können sich erst wieder entspannen, wenn die Lagerwelle 7 vollständig in die Lageröffnung 17 eingeschoben ist, die Rastvorsprünge 14 übergreifen dann die geradlinigen Abschnitte 20, 21 der Lageröffnung 17 und verhindern ein Herausziehen der Lagerwelle 7 aus der Lageröffnung 17, ein Herausziehen ist dann gar nicht mehr oder nur unter Überwindung einer bestimmten Haltekraft möglich. Es handelt sich also um eine Schnappverbindung, die am Ende des Zusammenschiebens wirksam wird.

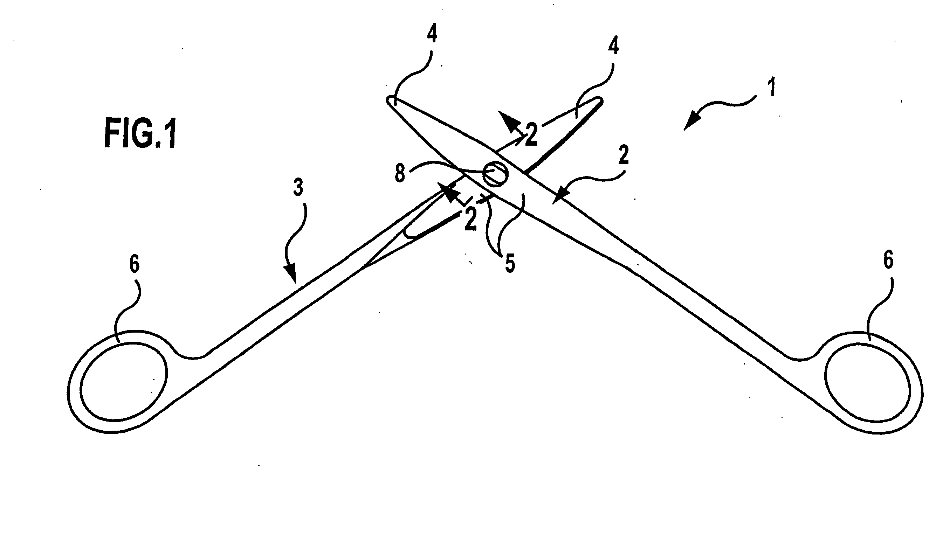

Die Lagerwelle 7 mit der Verbreiterung 8 und die Lageröffnung 17 sind so relativ zueinander angeordnet, daß die Verbreiterung 8 durch die Lageröffnung 17 hindurchgeschoben werden kann, wenn die beiden Scherenteile 2, 3 weit geöffnet sind, wie dies in Figur 1 dargestellt ist. Beim Verschwenken der beiden Scherenteile 2, 3 in Schließstellung gleitet die Unterseite 13 der Verbreiterung 8 auf zwei die Lageröffnung 17 unmittelbar anschießend an deren geradlinige Abschnitte 20, 21 sichelförmig umgebende Stützflächen 22, 23 auf, so daß dann dadurch ein Herausziehen der Lagerwelle 7 aus der Lageröffnung 17 sicher verhindert wird, es handelt sich um eine Bajonettverriegelung der beiden Scherenteile, die eine Verschwenkung der beiden Scherenteile bis in die vollständige Schließstellung ermöglicht, in der die beiden Scherenteile im wesentlichen parallel zueinander stehen (Figur 4).

Die Stützflächen 22 und 23 sind bei dem in der Zeichnung dargestellten Ausführungsbeispiel am Boden einer kreisförmigen Vertiefung 24 in dem Scherenteil 2 angeordnet, diese Vertiefung 24 umgibt die Lageröffnung 17 konzentrisch.

Die Stützflächen 22 und 23 sind gegenüber einer Ebene, die senkrecht auf der Schwenkachse der beiden Scherenteile steht und die nachstehend als Verschwenkebene bezeichnet wird, geringfügig geneigt, und zwar derart, daß die Scherenteile 2, 3 beim Verschwenken aus der in Figur 1 dargestellten Öffnungsstellung in die Schließstellung zunehmend gegeneinander gespannt werden. Bei dieser Schließbewegung gleiten die Unterseiten 13 der Verbreiterung 8 auf den Stützflächen 22, 23 entlang, die Stützflächen 22 und 23 können dabei über einen Winkel von 180° kontinuierlich ansteigend ausgebildet sein, es ist auch möglich, wie dies in Figur 6 dargestellt ist, daß die Stützflächen 22 und 23 einen ansteigenden und dann wieder einen abfallenden Bereich aufweisen, der in dem Bereich am höchsten ausgebildet ist, in dem die Unterseite 13 in der Schließstellung der beiden Scherenteile an den Stützflächen 22 und 23 anliegt.

Die Neigung der Stützflächen kann relativ gering sein, sie kann beispielsweise im Bereich zwischen ½° und 3° liegen, in Figur 6 ist diese Neigung nur aus Gründen der Deutlichkeit überhöht dargestellt.

Durch die vorgewölbte Ausbildung der Unterseite 13 ist sichergestellt, daß die Unterseite 13 an der Stützfläche 22 beziehungsweise 23 über den gesamten Verschwenkbereich linienförmig anliegt, es ist also eine genau definierte Zuordnung sichergestellt.

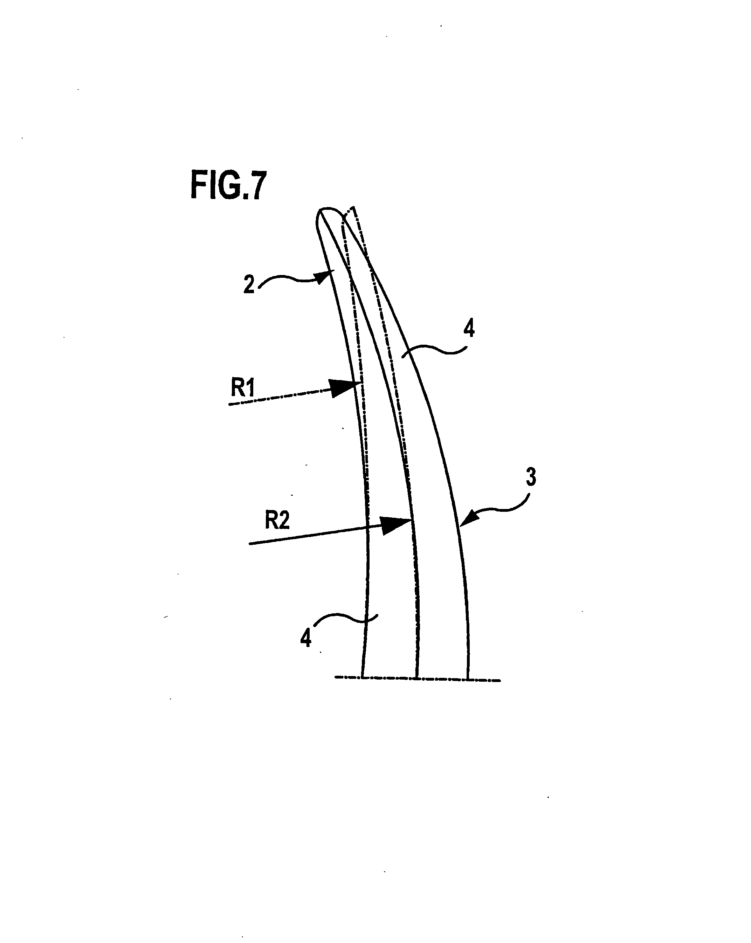

Die Scherenblätter 4 der beiden Scherenteile 2 und 3 sind beide aus der Verschwenkebene herausgebogen, wie dies aus der Darstellung der Figur 7 deutlich wird. Das innere Scherenteil 2 ist dabei mit einem Krümmungsradius R1 gebogen, das äußere Scherenteil 3 mit einem Krümmungsradius R2. Dabei ist der Krümmungsradius R1 größer als der Krümmungsradius R2, und zwar um etwa 10 bis 30 %. Dies führt dazu, daß sich das innere Scherenteil bei der Schließbewegung an dem äußeren Scherenteil abstützt, beide Scherenteile werden dabei so verformt, daß sie im Anlagebereich, das heißt im Bereich der Scherenblätter, kräftig gegeneinander gespannt werden. In Figur 7 ist zur Deutlichmachung in strichpunktierten Linien der Verlauf des unverformten inneren Scherenteils 2 dargestellt, durch die Anlage an dem äußeren Scherenteil 3 wird dieses Scherenteil so verformt, daß es den mit ausgezogenen Linien dargestellten Verlauf erhält, und durch diese Verformung werden beide Scherenteile 2, 3 zuverlässig elastisch gegeneinander gespannt, diese zusätzliche Spannung der Scherenteile überlagert sich noch der Spannung, die bei der Schließbewegung der Scherenteile dadurch erzeugt wird, daß die Unterseite 13 an den ansteigenden Stützflächen 22, 23 anliegend geführt wird und dadurch die beiden Scherenteile 2, 3 im Lagerbereich 5 gegeneinander spannt.

Die in der Zeichnung dargestellte Schere wird vorzugsweise aus einem sterilisierbaren Kunststoffmaterial hergestellt, welches eine Eigenelastizität aufweist, beispielsweise aus einem strahlen-, gas- oder dampfsterilisierbaren Kunststoff, insbesondere aus einem Gamma- oder ETO-(Ethylenoxid)-sterilisierbaren Kunststoff wie zum Beispiel PPA (Polyphenylamid). Die beschriebene Ausgestaltung ermöglicht es, auch Kunststoffmaterialien zu verwenden, die ein Langzeitkriechen zeigen. Diese Scheren werden dann vorzugsweise in der in Figur 1 dargestellten Öffnungsstellung aufbewahrt und erst bei Benutzung in die Schließstellung verschwenkt, so daß sichergestellt ist, daß erst bei der Benutzung die beiden Scherenteile gegeneinander gespannt werden. In diesem kurzen Benutzungszeitraum ergeben sich keine Verformungen durch unerwünschtes Kriechen des Kunststoffmaterials, dies könnte allenfalls dann erfolgen, wenn die Scheren lange Zeit in der Schließstellung aufbewahrt werden, und dies kann durch die beschriebene Lagerung in der Offenstellung vermieden werden.

## Patentansprüche

1. Schere (1) für medizinische Zwecke mit zwei in einem Schluß schwenkbar aneinander gelagerten, jeweils ein Scherenbiatt (4) umfassenden Scherenteilen (2, 3); wobei die beiden Scherenteile (2, 3) oder fest mit ihnen verbundene Teile (8) Spannelemente (13; 22, 23) aufweisen, die beim Verschwenken der Scherenteile (2, 3) aneinander anliegend relativ zueinander bewegt werden und die so geformt sind, daß die beiden Scherenteile (2, 3) beim Schließen aus einer Ruhestellung, in der die beiden Scherenblätter (4) in Richtung der Schwenkachse nicht oder nur wenig gegeneinander gespannt sind, in eine Spannstellung gelangen, in der die beiden Scherenblätter (4) in Richtung ihrer Schwenkachse einander angenähert und **dadurch** gegeneinander gespannt werden, wobei die Spannelemente eine gegenüber einer senkrecht auf der Schwenkachse stehenden Verschwenkebene geneigte bahnförmige Stützfläche (22, 23) und einen daran anliegenden Bahnfolger (13) umfassen, **dadurch gekennzeichnet, daß** die bahnförmige Stützfläche (22, 23) am Rande einer Lageröffnung (17) eines Scherenteils (2) und der Bahnfolger (13) an einer diese Lageröffnung (17) durchsetzenden Lagerwelle (7) des anderen Scherenteils (3) angeordnet sind.

2. Schere (1) für medizinische Zwecke mit zwei in einem Schluß schwenkbar aneinander gelagerten, jeweils ein Scherenblatt (4) umfassenden Scherenteilen (2, 3), wobei die beiden Scherenteile (2, 3) oder fest mit ihnen verbundene Teile (8) Spannelemente (13; 22, 23) aufweisen, die beim Verschwenken der Scherenteile (2, 3) aneinander anliegend relativ zueinander bewegt werden und die so geformt sind, daß die beiden Scherenteile (2, 3) beim Schließen aus einer Ruhestellung, in der die beiden Scherenblätter (4) in Richtung der Schwenkachse nicht oder nur wenig gegeneinander gespannt sind, in eine Spannstellung gelangen, in der die beiden Scherenblätter (4) in Richtung ihrer Schwenkachse einander angenähert und **dadurch** gegeneinander gespannt werden, wobei die Spannelemente eine gegenüber einer senkrecht auf der Schwenkachse stehenden Verschwenkebene geneigte bahnförmige Stützfläche (22, 23) und einen daran anliegenden Bahnfolger (13) umfassen, **dadurch gekennzeichnet, daß** der Bahnfolger am Rande einer Lageröffnung eines Scherenteils und die bahnförmige Stützfläche an einer diese Lageröffnung durchsetzenden Lagerwelle des anderen Scherenteils angeordnet sind.

3. Schere nach einem der voranstehenden Ansprüche, **dadurch gekennzeichnet, daß** der Bahnfolger (13) vorgewölbt ausgebildet ist, so daß er punkt- oder linienförmig an der Stützfläche (22, 23) anliegt.

4. Schere nach einem der voranstehenden Ansprüche, **dadurch gekennzeichnet, daß** ein Scherenteil (2) eine unrunde Lageröffnung (17) aufweist, durch die eine Lagerwelle (7) des anderen Scherenteils (3) hindurchragt, daß die Lagerwelle (7) an ihrem dem anderen Scherenteil (3) abgewandten Ende eine kopfförmige, unrunde Verbreiterung (8) trägt, die bei geöffneten Scherenteilen (2, 3) durch die unrunde Lageröffnung (17) hindurchpaßt und die beim Verschwenken der Scherenteil (2, 3) in Schließrichtung mit ihrer Unterseite (13) den Rand der Lageröffnung (17) überdeckt und damit ein Herausgleiten der Lagerwelle (7) aus der Lageröffnung (17) verhindert.

5. Schere nach Anspruch 4, **dadurch gekennzeichnet, daß** der Rand (22, 23) der Lageröffnung (17) und die Unterseite (13) der Verbreiterung (8) die beiden Spannelemente ausbilden.

6. Schere nach einem der Ansprüche 4 oder 5, **dadurch gekennzeichnet, daß** an der Lageröffnung (17) und der Lagerwelle (7) diametral einander gegenüberliegend je zwei bahnförmige Stützflächen (22, 23) beziehungsweise Bahnfolger (13) angeordnet sind.

7. Schere nach einem der voranstehenden Ansprüche, **dadurch gekennzeichnet, daß** die Neigung der bahnförmigen Stützfläche (22, 23) gegenüber der Verschwenkebene zwischen ½° und 3° liegt.

8. Schere nach einem der Ansprüche 4 bis 7, **dadurch gekennzeichnet, daß** die Lageröffnung (17) und/oder die Lagerwelle (7) elastische Rastelemente (14) tragen, die bei geöffneten Scherenteilen (2, 3) durch elastische Verformung ein Einschieben der Lagerwelle (7) in die Lageröffnung (17) ermöglichen, die jedoch nach dem Einschieben der Lagerwelle (7) in die Lageröffnung (17) elastisch entspannt ein Herausziehen der Lagerwelle (7) aus der Lageröffnung (17) verhindern oder erschweren.

9. Schere nach Anspruch 8, **dadurch gekennzeichnet, daß** die Rastelemente als seitliche Vorsprünge (14) an der Verbreiterung (8) der Lagerwelle (7) ausgebildet sind.

10. Schere nach Anspruch 9, **dadurch gekennzeichnet, daß** die Vorsprünge (14) seitliche Aufgleitflächen(15) tragen, an die sich ein Rücksprung (13, 16) anschließt.

11. Schere nach einem der voranstehenden Ansprüche, **dadurch gekennzeichnet, daß** mindestens eines der beiden Scherentelle (2, 3) im Bereich seines Scherenblattes (4) elastisch ausgebildet ist und daß mindestens eines der beiden Scherenteile (2, 3) aus der Verschwenkebene herausgebogen ist.

12. Schere nach Anspruch 11, **dadurch gekennzeichnet, daß** die Scherenteile (2, 3) im Bereich ihrer Scherenblätter (4) elastisch ausgebildet und aus der Verschwenkebene herausgebogen sind und daß der Biegeradius des inneren Scherenteits (2) größer ist als der des äußeren Scherenteils (3).

13. Schere nach Anspruch 11, **dadurch gekennzeichnet, daß** der Biegeradius des inneren Scherenteils (2) 10 bis 30 % größer ist als der des äußeren Scherenteils (3).

## Claims

1. Scissors (1) for medical purposes comprising two scissor parts (2, 3) pivotally mounted on one another at a joint and each comprising a scissor blade (4), wherein the two scissor parts (2, 3) or parts (8) securely connected to them have biasing elements (13; 22, 23), said elements abutting on one another during their movement relative to one another when the scissor parts (2, 3) are pivoted and being shaped such that during closure the two scissor parts (2, 3) pass from a rest position, the two scissor blades (4) not being biased or only slightly against one another in the direction of the pivot axis in said rest position, into a biasing position, the two scissor blades (4) being brought closer to one another in the direction of their pivot axis and thereby being biased against one another in said biasing position, wherein the biasing elements comprise a web-like supporting surface (22, 23) inclined in relation to a plane of pivoting extending at right angles to the pivot axis and a path follower (13) abutting thereon, **characterized in that** the web-like supporting surface (22, 23) is arranged at the edge of a bearing opening (17) of one scissor part (2) and the path follower (13) on a bearing shaft (7) of the other scissor part (3) passing through this bearing opening (17).

2. Scissors (1) for medical purposes comprising two scissor parts (2, 3) pivotally mounted on one another at a joint and each comprising a scissor blade (4), wherein the two scissor parts (2, 3) or parts (8) securely connected to them have biasing elements (13; 22, 23), said elements abutting on one another during their movement relative to one another when the scissor parts (2, 3) are pivoted and being shaped such that during closure the two scissor parts (2, 3) pass from a rest position, the two scissor blades (4) not being biased or only slightly against one another in the direction of the pivot axis in said rest position, into a biasing position, the two scissor blades (4) being brought closer to one another in the direction of their pivot axis and thereby being biased against one another in said biasing position, wherein the biasing elements comprise a web-like supporting surface (22, 23) inclined in relation to a plane of pivoting extending at right angles to the pivot axis and a path follower (13) abutting thereon, **characterized in that** the path follower is arranged at the edge of a bearing opening of one scissor part and the web-like supporting surface on a bearing shaft of the other scissor part passing through this bearing opening.

3. Scissors as defined in one of the preceding claims, **characterized in that** the path follower (13) is of a convex design so that it abuts on the supporting surface (22, 23) in a punctiform or linear manner.

4. Scissors as defined in any one of the preceding claims, **characterized in that** one scissor part (2) has a non-circular bearing opening (17), a bearing shaft (7) of the other scissor part (3) projecting through said opening, that the bearing shaft (7) bears a head-like, non-circular enlarged portion (8) at its end facing away from the other scissor part (3), said enlarged portion passing through the non-circular bearing opening (17) when the scissor parts (2, 3) are open and covering the edge of the bearing opening (17) with its underside (13) when the scissor parts (2, 3) are pivoted in the closing direction and thereby preventing the bearing shaft (7) from sliding out of the bearing opening (17).

5. Scissors as defined in claim 4, **characterized in that** the edge (22, 23) of the bearing opening (17) and the underside (13) of the enlarged portion (8) form the two biasing elements.

6. Scissors as defined in one of claims 4 or 5, **characterized in that** a respective two path-like supporting surfaces (22, 23) and path followers (13) located diametrically opposite one another are arranged at the bearing opening (17) and on the bearing shaft (7), respectively, or on the bearing shaft (7) and at the bearing opening (17), respectively.

7. Scissors as defined in any one of the preceding claims, **characterized in that** the inclination of the path-like supporting surface (22, 23) in relation to the plane of pivoting is between ½° and 3°.

8. Scissors as defined in any one of claims 4 to 7, **characterized in that** the bearing opening (17) and/or the bearing shaft (7) bear elastic detent elements (14) facilitating insertion of the bearing shaft (7) into the bearing opening (17) when the scissor parts (2, 3) are open due to elastic deformation but preventing or making more difficult any withdrawal of the bearing shaft (7) out of the bearing opening (17) when the elastic tension in them is released after the insertion of the bearing shaft (7) into the bearing opening (17).

9. Scissors as defined in claim 8, **characterized in that** the detent elements are designed as lateral projections (14) on the enlarged portion (8) of the bearing shaft (7).

10. Scissors as defined in claim 9, **characterized in that** the projections (14) bear lateral sliding surfaces (15), a shoulder (13, 16) adjoining said surfaces.

11. Scissors as defined in any one of the preceding claims, **characterized in that** at least one of the two scissor parts (2, 3) is of an elastic design in the area of its scissor blade (4) and that at least one of the two scissor parts (2, 3) is bent out of the plane of pivoting.

12. Scissors as defined in claim 11, **characterized in that** the scissor parts (2, 3) are of an elastic design in the area of their scissor blades (4) and are bent out of the plane of pivoting and that the radius of bend of the inner scissor part (2) is greater than that of the outer scissor part (3).

13. Scissors as defined in claim 11, **characterized in that** the radius of bend of the inner scissor part (2) is 10 to 30 % greater than that of the outer scissor part (3).

## Revendications

1. Ciseaux (1) à usage médical, comportant deux branches (2, 3), montées pivotantes l'une par rapport à l'autre dans une zone d'assemblage et munies chacune d'une lame de coupe (4), les deux branches (2, 3) ou des éléments (8) solidaires de celles-ci comportant des éléments de serrage (13 ; 22, 23), qui au moment du pivotement des branches (2, 3) se déplacent dans un mouvement relatif en étant en appui les uns contre les autres et qui sont formés de telle sorte que les deux branches (2, 3) au moment de la fermeture hors d'une position de repos, dans laquelle les deux lames de coupe (4) ne sont pas serrées l'une contre l'autre dans la direction de l'axe de pivotement ou le sont seulement faiblement, parviennent dans une position de serrage, dans laquelle les deux lames de coupe (4) sont rapprochées l'une de l'autre dans la direction de leur axe de pivotement et, de ce fait, sont serrées l'une contre l'autre, les éléments de serrage comportant une face d'appui (22, 23) en forme de bande, inclinée par rapport à un plan de pivotement perpendiculaire à l'axe de pivotement, et un suiveur de trajectoire (13) adjacent à ladite face d'appui, **caractérisés en ce que** la face d'appui (22, 23) en forme de bande est agencée sur le bord d'un orifice de réception (17) dans une branche (2) et le suiveur de trajectoire (13) est agencé sur un pivot (7) de l'autre branche (3), passant à travers cet orifice de réception (17).

2. Ciseaux (1) à usage médical, comportant deux branches (2, 3), montées pivotantes l'une par rapport à l'autre dans une zone d'assemblage et munies chacune d'une lame de coupe (4), les deux branches (2, 3) ou des éléments (8) solidaires de celles-ci comportant des éléments de serrage (13 ; 22, 23), qui au moment du pivotement des branches (2, 3) se déplacent dans un mouvement relatif en étant en appui les uns contre les autres et qui sont formés de telle sorte que les deux branches (2, 3) au moment de la fermeture hors d'une position de repos, dans laquelle les deux lames de coupe (4) ne sont pas serrées l'une contre l'autre dans la direction de l'axe de pivotement ou le sont seulement faiblement, parviennent dans une position de serrage, dans laquelle les deux lames de coupe (4) sont rapprochées l'une de l'autre dans la direction de leur axe de pivotement et, de ce fait, sont serrées l'une contre l'autre, les éléments de serrage comportant une face d'appui (22, 23) en forme de bande, inclinée par rapport à un plan de pivotement perpendiculaire à l'axe de pivotement, et un suiveur de trajectoire (13) adjacent à ladite face d'appui, **caractérisés en ce que** le suiveur de trajectoire est agencé sur le bord d'un orifice de réception dans une branche et la face d'appui en forme de bande est agencée sur un pivot de l'autre branche, passant à travers cet orifice de réception.

3. Ciseaux selon l'une quelconque des revendications précédentes, **caractérisés en ce que** le suiveur de trajectoire (13) est réalisé avec une surface convexe, de manière à venir en appui par points ou par lignes contre la face d'appui (22, 23).

4. Ciseaux selon l'une quelconque des revendications précédentes, **caractérisés en ce qu'**une branche de ciseaux (2) comporte un orifice de réception (17) non circulaire, à travers lequel passe un pivot (7) de l'autre branche (3), **en ce que** le pivot (7) sur son extrémité opposée à l'autre branche (3) comporte un élargissement (8) non circulaire en forme de tête, qui passe à travers l'orifice de réception (7) non circulaire lorsque les branches (2, 3) sont ouvertes et qui, au moment du pivotement des branches (2, 3) dans la direction de fermeture, couvre avec sa face inférieure (13) le bord de l'orifice de réception (17) et empêche ainsi le pivot (7) de glisser hors de l'orifice de réception (17).

5. Ciseaux selon la revendication 4, **caractérisés en ce que** le bord (22, 23) de l'orifice de réception (17) et la face inférieure (13) de l'élargissement (8) forment les deux éléments de serrage.

6. Ciseaux selon la revendication 4 ou 5, **caractérisés en ce que** respectivement deux faces d'appui (22, 23) en forme de bande et deux suiveurs de trajectoire (13), diamétralement opposés, sont réalisés sur l'orifice de réception (17) et le pivot (7).

7. Ciseaux selon l'une quelconque des revendications précédentes, **caractérisés en ce que** l'inclinaison de la face d'appui (22, 23) en forme de bande par rapport au plan de pivotement se situe entre 1/2° et 3°.

8. Ciseaux selon l'une quelconque des revendications 4 à 7, **caractérisés en ce que** l'orifice de réception (17) et/ou le pivot (7) comportent des éléments d'arrêt (14) élastiques qui, dans la position ouverte des branches (2, 3), permettent moyennant une déformation élastique l'introduction du pivot (7) dans l'orifice de réception (17), mais lesquels, après l'introduction du pivot (7) dans l'orifice de réception (17) se détendent élastiquement et empêchent ou rendent difficile une extraction du pivot (7) hors de l'orifice de réception (17).

9. Ciseaux selon la revendication 8, **caractérisés en ce que** les éléments d'arrêt sont conçus sous forme de saillies (14) latérales sur l'élargissement (8) du pivot (7).

10. Ciseaux selon la revendication 9, **caractérisés en ce que** les saillies (14) sont munies de faces de glissement (15) latérales, qui se prolongent par un épaulement (13, 16).

11. Ciseaux selon l'une quelconque des revendications précédentes, **caractérisés en ce qu'**au moins l'une des deux branches (2, 3) est réalisée flexible dans la zone de sa lame de coupe (4) et **en ce qu'**au moins l'une des deux branches (2, 3) est cintrée hors du plan de pivotement.

12. Ciseaux selon la revendication 11, **caractérisés en ce que** les branches (2, 3) dans la zone de leurs lames de coupe (4) sont conçues de manière flexible et sont cintrées hors du plan de pivotement et **en ce que** le rayon de flexion de la branche intérieure (2) est plus grand que celui de la branche extérieure (3).

13. Ciseaux selon la revendication 11, **caractérisés en ce que** le rayon de flexion de la branche intérieure (2) est 10 à 30 % supérieur à celui de la branche extérieure (3).
